(19) Europäisches Patentamt
European Patent Office
Office européen des brevets

(11) EP 4 648 017 A1

(12) EUROPEAN PATENT APPLICATION
published in accordance with Art. 153(4) EPC

(43) Date of publication:
12.11.2025 Bulletin 2025/46

(51) International Patent Classification (IPC):
G06T 17/00 (2006.01)    G06V 10/82 (2022.01)

(21) Application number: 23933567.2

(52) Cooperative Patent Classification (CPC):
G06T 17/00; G06V 10/42; G06V 10/82

(22) Date of filing: 16.05.2023

(86) International application number:
PCT/CN2023/094488

(87) International publication number:
WO 2024/216692 (24.10.2024 Gazette 2024/43)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 18.04.2023  CN 202310408673

(71) Applicant: Enchannel Medical Guangzhou Inc.
Guangzhou, Guangdong 510700 (CN)

(72) Inventors:
• KONG, Fanwei
  Guangzhou, Guangdong 510700 (CN)
• ZHU, Min
  Guangzhou, Guangdong 510700 (CN)
• SONG, Qing
  Guangzhou, Guangdong 510700 (CN)
• FENG, Jun
  Guangzhou, Guangdong 510700 (CN)

(74) Representative: Manitz Finsterwald
Patent- und Rechtsanwaltspartnerschaft mbB
Martin-Greif-Straße 1
80336 München (DE)

(54) **METHOD FOR CONSTRUCTING THREE-DIMENSIONAL ANATOMICAL MODEL OF HEART, AND SYSTEM FOR THREE-DIMENSIONAL MAPPING OF HEART**

(57)     Disclosed are a method for constructing a 3D cardiac anatomical model and a 3D cardiac mapping system. The method includes: obtaining an atrial template model and point cloud data of the atrium to be modeled, the data comprising positional coordinate information of partial feature points within said atrium; using an affine transformation network to map the partial feature points to the atrial template model; inputting the point cloud data mapped to the atrial template model into a prediction network to predict the displacements of the control feature points in the atrial template model, and deforming the atrial template model based on said displacements to obtain a 3D anatomical model of said atrium. This approach enables fully automated construction of the 3D atrial anatomical model, by means of the positional coordinate information of a portion of the feature points of an atrium to be modeled, and utilizing a trained neural network, construct a three-dimensional anatomical model of the atrium to be modeled; moreover, human intervention is not required.

FIG. 1

# Description

## TECHNICAL FIELD

The present disclosure relates to the field of electrocardiography, and more particularly to methods for constructing a three-dimensional (3D) cardiac anatomical model and to 3D cardiac mapping systems.

## BACKGROUND OF THE DISCLOSURE

In a cardiac mapping system, an electrode catheter is placed within a heart. The system determines the location of the electrode catheter via magnetic or electric fields. By manipulating the electrode catheter, its electrodes are brought into close contact with the endocardial wall to acquire spatial points. After comprehensively sampling positional coordinates across the entire atrial endocardial surface, dense point cloud data is obtained, which is then used to construct a three-dimensional (3D) atrial anatomical model. However, certain atrial regions, such as the left and right pulmonary veins and atrial appendages, are difficult for the electrode catheter to access. This significantly increases the difficulty of catheter manipulation and may cause inaccurate 3D anatomical models due to insufficient point density in these regions. Furthermore, direct construction of the 3D atrial anatomical model from the point cloud data often requires manual modification by specialized personnel. Examples include highlighting the positions of the pulmonary veins, highlighting the position of the ridge/crista between the left superior pulmonary vein and the left atrial appendage, and creating openings/orifices at the mitral and tricuspid valve positions.

In summary, current methods for constructing 3D cardiac anatomical models still face challenges including but not limited to: reliance on extensive dense sampling of data points, model inaccuracies, and dependence on manual adjustments by specialized personnel.

## SUMMARY OF THE DISCLOSURE

To address the aforementioned challenges, the present disclosure provides methods for constructing a 3D cardiac anatomical model as well as 3D cardiac mapping systems in accordance with embodiments of the present disclosure.

According to a first aspect of the present disclosure, a method for constructing a three-dimensional cardiac anatomical model is provided in some embodiments, comprising:

obtaining an atrial template model, wherein the atrial template model is a three-dimensional mesh model of a predetermined atrial boundary and is provided with a plurality of predetermined control feature points, and the atrial template model deforms based on displacement of the control feature points;

obtaining point cloud data of an atrium to be modeled within a heart, wherein the point cloud data of the atrium to be modeled comprises positional coordinate information of partial feature points within the atrium to be modeled;

mapping the partial feature points in the atrium to be modeled to the atrial template model by using an affine transformation network, thereby obtaining point cloud data mapped to the atrial template model;

inputting the point cloud data mapped to the atrial template model into a prediction network to predict displacements of the control feature points in the atrial template model; and

obtaining a three-dimensional anatomical model of the atrium to be modeled by deforming the atrial template model based on the displacement of the control feature points.

According to a second aspect of the present disclosure, a three-dimensional cardiac mapping system is provided in some embodiments, comprising:

an acquisition device, configured to obtain point cloud data of an atrium to be modeled within a heart;

a processor, configured to execute the method for constructing a three-dimensional cardiac anatomical model according to any one of embodiments mentioned above, so as to output a three-dimensional anatomical model of the atrium to be modeled; and

a display device, configured to display the three-dimensional anatomical model of the atrium to be modeled.

According to a third aspect of the present disclosure, a computer-readable storage medium is provided in some embodiments, wherein the medium stores a program that, when executed by a processor, is capable of implementing the method according to any one of embodiments mentioned above.

According to the method for constructing a three-dimensional cardiac anatomical model and the three-dimensional cardiac mapping system according to the aforementioned embodiments of the present disclosure, an atrial template model and point cloud data of the atrium to be modeled are obtained. The point cloud data comprises positional coordinate information of partial feature points within the atrium to be modeled. The partial feature points in the atrium to be modeled are mapped to the atrial template model using an affine transformation network. The point cloud data mapped to the atrial template model is inputted into a prediction network to predict displacements of the control feature points in the atrial template model. The atrial template model is then deformed based on the displacements of the control feature points, thereby obtaining the three-dimensional anatomical model of the atrium to be modeled. Accordingly, the present disclosure can construct the three-dimensional anatomical model of the atrium to be modeled based on positional coordinate information of partial feature points of the atrium to be modeled by using a trained neural network, enabling fully automated construction of the three-dimensional anatomical model of the atrium without manual intervention.

## BRIEF DESCRIPTION OF THE DRAWINGS

FIG. 1 is a flowchart of a method for constructing a three-dimensional cardiac anatomical model based on point cloud data according to some embodiments of the present disclosure;

FIG. 2 is a specific flowchart of a method for constructing a three-dimensional cardiac anatomical model according to some embodiments of the present disclosure; and

FIG. 3 is a structural schematic diagram of a three-dimensional cardiac mapping system according to some embodiments of the present disclosure.

## DETAILED DESCRIPTION

Specific embodiments of the present disclosure will now be described in detail with reference to the accompanying drawings. Similar or related components in different embodiments are labeled with associated reference numerals. The following embodiments include detailed descriptions to facilitate understanding of the present disclosure. However, those skilled in the art will readily recognize that certain features may be omitted under specific circumstances or substituted by other components, materials, or methods. In some instances, certain operations related to the present disclosure are not explicitly described or illustrated herein. This intentional exclusionis intentional to avoid obscuring the core technical solutions of the present disclosure. For those skilled in the art, a complete understanding of these operations can be attained through the descriptions provided in this specification and general technical knowledge in the art.

Additionally, the features, operations, or characteristics described in the specification may be combined in any suitable manner to form various embodiments. Similarly, steps or actions in the method descriptions may be reordered or modified in ways that would be obvious to those skilled in the art. Therefore, the sequences presented in the specification and drawings are intended solely to clarify the description of specific embodiments and do not imply mandatory orderings, unless explicitly stated that a particular sequence is required.

The numerical designations assigned to components in this specification, such as 'first,' 'second,' or similar ordinal terms, serve solely to distinguish described objects and carry no inherent sequential or technical implications. Furthermore, the terms 'connected' and 'coupled' as used herein encompass both direct and indirect connection (coupling), unless explicitly stated otherwise. The following are the references involved in the embodiments of the present disclosure:

Reference 1: Marching cubes: A high resolution 3D surface construction algorithm. Lorensen, W.E., & Cline, H.E. (1987). Proceedings of the 14th annual conference on Computer graphics and interactive techniques.;
Reference 2: Linear subspace design for real-time shape deformation. Wang, Y., Jacobson, A., Barbi č, J., & Kavan, L. (2015). ACM Transactions on Graphics (TOG), 34, 1 - 11.;
Reference 3: Learning Whole Heart Mesh Generation From Patient Images for Computational Simulations. Kong, F., & Shadden, S.C. (2022). IEEE Transactions on Medical Imaging, 42, 533-545.;
Reference 4: PointNet++: Deep Hierarchical Feature Learning on Point Sets in a Metric Space. Qi, C., Yi, L., Su, H., & Guibas, L.J. (2017). NIPS. and
Reference 5: PointNet: Deep Learning on Point Sets for 3D Classification and Segmentation. Qi, C., Su, H., Mo, K., & Guibas, L.J. (2016). 2017 IEEE Conference on Computer Vision and Pattern Recognition (CVPR), 77-85.

In the embodiments of the present disclosure, a complete and relatively accurate 3D atrial anatomical model can be reconstructed based on a pre-constructed atrial template model and positional coordinate information of a limited number of collected intracardiac feature points.

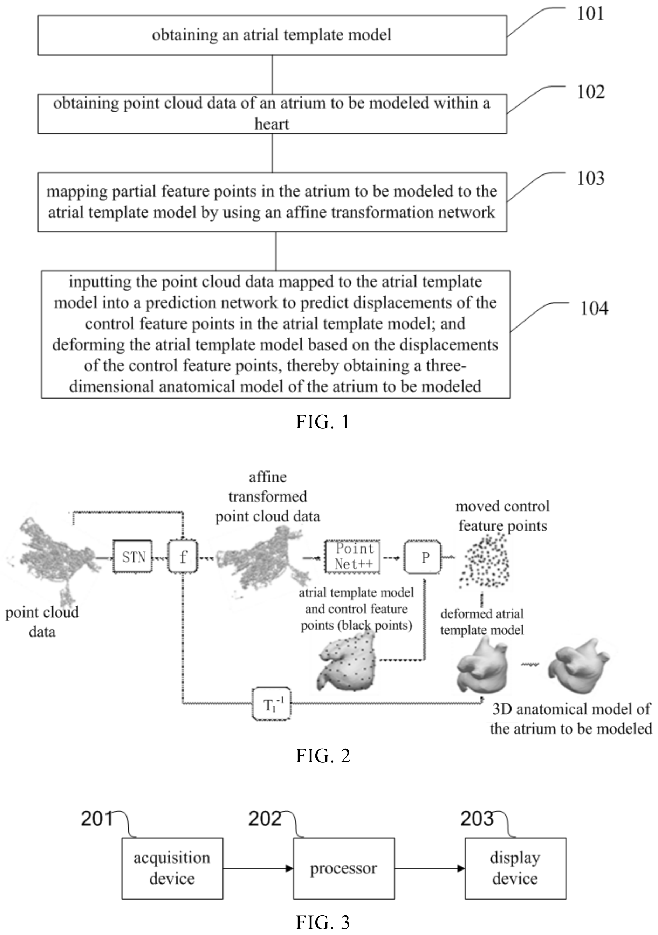

Referring to FIG. 1, a method for constructing a 3D cardiac anatomical model based on point cloud data (hereinafter also referred to as a 3D cardiac anatomical model construction method) is provided in some embodiments of the present disclosure. The 3D cardiac anatomical model construction method includes steps 101 to 104, which will be described in detail below.

Step 101: obtaining an atrial template model, wherein the atrial template model is a three-dimensional mesh model of a predetermined atrial boundary and is provided with a plurality of predetermined control feature points, and the atrial template model is deformed in accordance with displacements of the control feature points.

In this embodiment, the atrial template model is a pre-constructed predetermined 3D atrial mesh model suitable for subsequent electrophysiological analysis. It can be constructed using various existing approaches. The following construction methods are provide:
A cardiac CT scan image is selected. The anatomical structures of the left and right atrium are segmented from this cardiac CT scan image, and the segmented anatomical structures are converted into a 3D mesh model. In this embodiment, the Marching cube method (Reference 1) may be employed to extract the isosurface to obtain a 3D mesh model representing atrial boundaries. This 3D mesh model serves as the atrial template model. On the mesh surface of the atrial template model, a set of key points needs to be selected as control feature points. These control feature points maintain a linear transformation relationship with all mesh vertices of the atrial template model, enabling the control feature points to control the mesh deformation of the atrial template model. Specifically, displacements of the control feature points and displacements of all mesh vertices of the atrial template model exhibit a linear transformation relationship, which may be computed using Biharmonic coordinates (Reference 2). In an embodiment, a linear transformation relationship (denoted as $WER^{(n \times c)}$) connecting the displacement of each control feature point to each mesh vertex may be calculated by solving an optimization problem involving the biharmonic equation. Here, n represents the number of mesh vertices of the atrial template model, c represents the number of control feature points in the atrial template model, and $R^{(n \times c)}$ denotes the mapping set between the mesh vertices of the atrial template model and the control feature points of the atrial template model. The mesh vertices of the atrial template model undergo smooth deformation following coordinate changes of the control feature points. Assuming the coordinates of the mesh vertices of the atrial template model are $PER^{(n \times 3)}$ (where $R^{(n \times 3)}$ represents a set of positional coordinate information of the mesh vertices in the atrial template model), and the coordinates of the control feature points are $Q \in R^{(c \times 3)}$ (where $R^{(c \times 3)}$ represents a set of positional coordinate information of the control feature points), then the coordinates of the mesh vertices and the coordinates of the control feature points satisfy $P = WQ$. If a matrix $S \in R^{(c \times n)}$ represents the selection of Q from P, it must satisfy the linear constraint $SP = Q$.

Additionally, regarding the selection of control feature points, multiple approaches may be employed depending on circumstances. For example, control feature points may be selected as uniformly distributed points across the mesh surface of the atrial template model, or points at anatomically significant locations of the atrium may be selected as control feature points, including but not limited to pulmonary vein ostia and the atrial roof.

Step 102: obtaining point cloud data of an atrium to be modeled within a heart. The point cloud data of the atrium to be modeled comprises positional coordinate information of partial feature points within the atrium to be modeled. The atrium to be modeled may be the left atrium or the right atrium of the heart. In this embodiment, electrode catheters and/or electrode patches are employed to collect positional coordinate information of feature points within the atrium to be modeled. The collection may be performed either by: exclusively using electrode catheters or combining electrode catheters with electrode patches. Electrode catheters refer to catheters comprising one or more electrodes. Positional coordinate information of intracardiac feature points is acquired by inserting the catheter into the cardiac chamber and moving the electrodes. Electrode patches determine spatial coordinates of catheter-carried electrodes by being attached to the chest or back of an object under examination, thereby enabling the electrode catheters to collect positional coordinate information of feature points with enhanced accuracy.

It should be noted that the feature points acquired from the atrium to be modeled in this embodiment constitute a limited number of feature points, which does not require operation of electrode catheter to perform intensive point-by-point mapping.

Step 103: mapping partial feature points in the atrium to be modeled to the atrial template model by using an affine transformation network, thereby obtaining point cloud data mapped to the atrial template model. The atrial template model and the point cloud data obtained in steps 101 and 102 are used as inputs to the affine transformation network, and the affine transformation network outputs point cloud data mapped to the atrial template model.

Before inputting the point cloud data into the affine transformation network, it is necessary to perform normalization processing on the point cloud data obtained in step 102. This involves subtracting the centroid coordinates of the point cloud data from the coordinates of each feature point in the point cloud data and then dividing by the maximum radius from the centroid to obtain normalized point cloud data. The normalized point cloud data is then fed into the affine transformation network. In this embodiment, a fully-connected neural network is employed to predict the affine transformation between the atrial model represented by the feature points in the point cloud data and the atrial template model, thereby enabling mapping of each feature point in the input point cloud data onto the atrial template model.

In an embodiment, mapping partial feature points in the atrium to be modeled to the atrial template model using the affine transformation network to obtain point cloud data mapped to the atrial template model includes:

performing normalization processing on the point cloud data of the atrium to be modeled;
inputting the normalized point cloud data into a fully-connected neural network to predict an affine transformation matrix between the atrium to be modeled and the atrial template model; and
mapping partial feature points in the atrium to be modeled to the atrial template model based on the predicted affine transformation matrix, thereby obtaining point cloud data mapped to the atrial template model.

Here, the affine transformation network includes ReLU activation functions and batch normalization layers. If the function represented by this affine transformation network is f, then f: f: $R^{(m \times 3)} \rightarrow R^q$, $\sigma = f_\theta$ (Q, $V_{init}$), where m represents the number of feature points in the point cloud data input to the affine transformation network, $R^{(m \times 3)}$ represents a set of point cloud data input to the affine transformation network, q represents the number of parameters required for the affine transformation, $R^q$ represents a set of parameters required for the affine transformation, $\sigma$ is an affine transformation parameter, and $\theta$ is the parameter of the affine transformation network. Generally, an arbitrary affine transformation requires 12 parameters. However, this number can be reduced according to requirements. For example, if the atrium represented by the acquired point cloud data is roughly aligned with the atrial template model in orientation, so that aligning it (roughly) only requires adjusting the size (i.e., scaling) of the atrium represented by the point cloud data, then only three scaling parameters (along the x, y, z axes) are needed, eliminating the need for rotational parameters. Finally, the output parameters from the affine transformation network are converted into a corresponding affine transformation matrix. This matrix is applied to map the feature points of the input point cloud data to the atrial template model.

Step 104: inputting the point cloud data mapped to the atrial template model into a prediction network to predict displacements of the control feature points in the atrial template model; and deforming the atrial template model based on the displacements of the control feature points, thereby obtaining a three-dimensional anatomical model of the atrium to be modeled.

In this embodiment, after the point cloud data is mapped to the atrial template model, it is input into a prediction network. This prediction network is used to predict the displacements of the control feature points in the atrial template model. By adding the displacements of the control feature points to the initial positions of the control feature points in the atrial template model, the atrial template model is deformed. Based on the deformed atrial template model and the initially input point cloud data, the 3D anatomical model of the atrium to be modeled is obtained, expressed as: $V_p = (T_1^{-1}(WP_{init} + \Delta P)^T)^T$, where $T_1^{-1}$ is the inverse transformation of the affine transformation output by the affine transformation network, $V_p$ represents the mesh vertex coordinates of the 3D anatomical model of the atrium to be modeled, $\Delta P$ represents the displacements of the control feature points, and $P_{init}$ represents the initial positions of the control feature points.

In an embodiment, the prediction network may adopt a neural network similar to the PointNet++ structure (Reference 4). The PointNet++ neural network includes a plurality of point set abstraction modules. The process of inputting the point cloud data mapped to the atrial template model into the prediction network to predict the displacements of the control feature points in the atrial template model includes:

inputting the point cloud data mapped to the atrial template model into a first point set abstraction module, and then inputting the data output by the first point set abstraction module into a second point set abstraction module, and continuing this sequential processing through subsequent module. The data output by a last point set abstraction module comprises the predicted displacements of the control feature points in the atrial template model.

Each point set abstraction module performs operations as follows:
randomly sampling k points from the input data and taking each sampled point as a centroid point, and selecting m points within a spherical region having a specified radius centered on each centroid point; taking m points corresponding to each sampled point as a point set; extracting the feature information for each point set (in this embodiment, the feature information may be extracted by the approach provided in Reference 5), and obtaining global feature information corresponding to each sampled point by using a max pooling operation; and outputting the sampled k points and the global feature information corresponding to each sampled point.

It should be noted that the prediction network in this embodiment differs from a typical PointNet++ structured neural network in its use of farthest point sampling. A typical PointNet++ network employed farthest point to randomly sample points from the input point cloud data. Conversely, the prediction network in this embodiment uses farthest point sampling to select a specified number of centroid points in the atrial template model, which are then used to group the input feature points. Since the centroid points can be pre-selected in the atrial template model, the calculation for farthest point sampling is avoided during both training and testing phases, saving computational time. Furthermore, directly sampling centroid points on the atrial template model (rather than in the input data) ensures that the final displacement predictions are centered on the control feature points. This approach significantly improves prediction accuracy.

In summary, the method for constructing a 3D cardiac anatomical model provided in some embodiments of the present disclosure requires the use of an affine transformation network and a prediction network. Accordingly, before using the affine transformation network and the prediction network, they need to be trained first. The training process may be as follows:

(1) Constructing training data: The training data comprises two components: one being the ground truth of the 3D atrial anatomical model, and the other being the point cloud data used for training. For acquisition of the ground truth of the 3D atrial anatomical model, multiple cardiac CT images may be processed using existing machine learning methods (Reference 3) to extract a 3D mesh-represented atrial anatomical model from the multiple cardiac CT images. These extracted 3D atrial anatomical models serves as the training ground truth. For the point cloud data used for training, such data may be randomly generated by simulating the process of electrodes acquiring points within the atrium. Specifically, since electrode shapes of different configurations (linear, mesh) are analytically expressible, electrode movement trajectories within the atrium are simulated, whereby electrode coordinates are recorded in real-time to generate point cloud data. The electrode movement trajectories may include translational and rotational motion to pulmonary vein ostia and other key atrial sites, while stochastic elements are incorporated into the trajectories to simulate operational uncertainties.

All training data is proportionally divided into a training set, a validation set and a test set. The training set is used to train the neural network and optimize its parameters, the validation set serves for adjusting hyperparameters of the deep learning method, and the test set is employed to validate the accuracy of point cloud meshing. Concurrently, the training data in embodiments of the present disclosure may incorporate actually collected atrial point cloud data to additionally validate the trained neural network.

(2) Performing parameter optimization for the affine transformation network and the prediction network using the training data.

For the optimization of the affine transformation network, the affine transformation network is a fully-connected neural network trained using the backpropagation algorithm based on the first loss function. The first loss function is defined by the following expression:

$$L_1 = \| V_{init} - (T_1 V^T_{groundtruth})^T \|^2_2,$$

where $L_1$ represents the value of the first loss function, $V_{init}$ represents a matrix corresponding to the positional coordinate information of each mesh vertex in the atrial template model, $T_1$ represents the affine transformation matrix, $V_{groundtruth}$ represents a matrix corresponding to coordinate ground truth values of mesh vertices in the 3D network corresponding to the atrium to be modeled, and $\| \|^2_2$ represents a squared Euclidean norm.

For the optimization of the prediction network, the prediction network is trained using the backpropagation algorithm based on the second loss function. The second loss function is defined by the following expression:

$$L_2 = \| V_p - V_{groundtruth} \|^2_2,$$

where $L_2$ represents the value of the second loss function, $V_p$ represents a matrix corresponding to coordinate prediction values of mesh vertices in the 3D mesh model of the atrium to be modeled, $V_{groundtruth}$ represents a matrix corresponding to coordinate ground truth values of mesh vertices in the 3D anatomical model corresponding to the atrium to be modeled, and $\| \|^2_2$ represents the squared Euclidean norm.

A total loss function $L=L_1+L_2$ is constructed from the first loss function and the second loss function. The training is performable using the stochastic gradient descent method.

Referring to FIG. 2, in some embodiments of the present disclosure, the point cloud data of the atrium to be modeled and the atrial template model (STN) are inputted into an affine transformation network (f) for affine transformation. The affine transformation network (f) outputs the transformed point cloud data mapped to the atrial template model, which is then input into a prediction network (PointNet++). The prediction network (PointNet++) outputs predicted displacements ($\Delta P$) of the control feature points. Then, based on the initial positions of the control feature points in the atrial template model, moved positions of the control feature points are determined to obtain a deformed atrial template model. Finally, a final 3D anatomical model of the atrium to be modeled is obtained by applying an inverse affine transformation $T_1^{-1}$ to the deformed atrial template model, wherein the inverse transformation utilizes the initially collected point cloud data of the atrium to be modeled.

Referring to FIG. 3, a 3D cardiac mapping system is further provided in some embodiments of the present disclosure. The 3D cardiac mapping system includes: an acquisition device 201, a processor 202 and a display device 203. The acquisition device 201 is configured to obtain point cloud data of an atrium to be modeled within a heart. The processor 202 may be configured to execute the method for constructing a 3D cardiac anatomical model based on point cloud data according to any of the above embodiments. The display device 203 is configured to display the 3D atrial anatomical model of the atrium.

The acquisition device 201 may be an electrode catheter and/or an electrode patch. This device acquire positional coordinate information of the feature points in the atrium by using the electrode catheter alone, or by combining the electrode catheter with the electrode patch. The electrode catheter, configured with one or more electrodes, collects the positional coordinate information of the feature points in the atrium by inserting the catheter into the atrial chamber and subsequent electrode movement. The electrode patch, when attached to the chest or back of an object under examination, providing spatial localization for catheter-mounted electrodes. This configuration allows the electrode catheter to collect more accurate positional coordinate information of the feature points. The processor 202 comprises an integrated circuit chip possessing computational processing capabilities. The processor 202 may be a general-purpose processor (e.g., a central processing unit [CPU], a network processor [NP]) configured to execute method steps and implement logic blocks disclosed in the embodiments of the present disclosure. The general-purpose processor includes, but is not limited to, a microprocessor or any conventional processor. The display device 203 supports human-computer interaction functionalities, rendering both 3D atrial anatomical model and user command interfaces (e.g., touch-sensitive displays). Alternatively, the device 203 operates exclusively to display the 3D atrial anatomical model.

It should be understood that the configuration shown in FIG. 3 is exemplary only. The system may incorporate components beyond those illustrated and/or omit depicted components, or adopt alternative configurations. The components in FIG. 3 may be implemented in hardware, software, or combinations thereof. For example, the 3D cardiac mapping system may further include a memory (not shown) configured to: store acquired structured 3D image data of a target anatomy, and store computer programs executable by the processor 202 for implementing the method for constructing a 3D cardiac anatomical model disclosed in preceding method embodiments. Suitable memory types include, but are not limited to, random access memory (RAM), read only memory (ROM), programmable read-only memory (PROM), erasable programmable read-only memory (EPROM), electric erasable programmable read-only memory (EEPROM).

The present disclosure refers to various exemplary embodiments for illustrative purposes. However, those skilled in the art will recognize that modifications and alterations may be made to these embodiments without departing from the scope of the disclosure. For instance, individual operational steps and components for performing such steps may be implemented in diverse manners depending on specific applications or considerations of cost functions associated with system operations (e.g., one or more steps may be deleted, modified, or consolidated with other steps).

Moreover, as understood by those skilled in the art, the principles disclosed may be embodied in a computer program product stored on a non-transitory computer-readable storage medium preloaded with computer-readable program code. Any tangible, non-transitory computer-readable storage medium may be utilized, including but not limited to: magnetic storage devices (e.g., hard disks, floppy disks); optical storage devices (e.g., CD-ROMs, DVDs, Blu-ray discs); and flash memory devices. The computer program instructions may be loaded onto a general-purpose computer, special-purpose computer, or other programmable data processing apparatus to create a machine, such that the instructions executed on the computer or programmable apparatus produce means for implementing specified functions. These instructions may also reside in a computer-readable memory, directing the computer or programmable apparatus to operate in a defined manner, thereby forming an article of manufacture comprising functional implementation means. Furthermore, the computer program instructions may be executed on a computer or programmable data processing apparatus to generate a computer-implemented process, wherein the executed instructions provide steps for realizing the specified functionality, including but not limited to: technical improvements in data processing efficiency (e.g., optimized memory allocation); and enhanced accuracy in algorithmic execution (e.g., reduced error margins in machine learning models).

While the principles disclosed herein have been illustrated through various embodiments, it should be understood that structural configurations, material selections, and component proportions particularly suited to specific operational environments may be modified without departing from the scope and spirit of the disclosure. Such modifications, along with other adaptations or adjustments, shall be encompassed within the scope of the present disclosure.

The foregoing detailed description has been described with reference to various embodiments. However, those skilled in the art will recognize that modifications and variations may be made without departing from the scope of the disclosure. Accordingly, the description of the disclosure shall be interpreted in an illustrative rather than restrictive sense, and all such modifications are intended to be included within its scope. Similarly, discussions of advantages, alternative solutions to problems, and operational benefits associated with the embodiments are provided above. Nevertheless, benefits, advantages, solutions to problems, and any elements that may produce such effects or render them more explicit shall not be construed as critical, required, or essential. Furthermore, the term 'coupled' and its derivatives encompass physical connections (e.g., mechanical joints), electrical connections (e.g., circuit interconnects), magnetic linkages (e.g., inductive coupling), optical interfaces (e.g., fiber-optic alignment), communication channels (e.g., wireless protocols), functional integrations (e.g., software APIs), and any other form of association that achieves operational interaction.

Those skilled in the art will recognize that numerous modifications to the details of the above-described embodiments may be made without departing from the fundamental principles of the disclosed subject matter. Accordingly, the scope of the present disclosure shall be determined solely by the claims and their legal equivalents.

**Claims**

1. A method for constructing a three-dimensional cardiac anatomical model based on point cloud data, comprising:

obtaining an atrial template model, wherein the atrial template model is a three-dimensional mesh model of a predetermined atrial boundary and is provided with a plurality of predetermined control feature points, and the atrial template model deforms based on displacement of the control feature points;

obtaining point cloud data of an atrium to be modeled within a heart, wherein the point cloud data of the atrium to be modeled comprises positional coordinate information of partial feature points within the atrium to be modeled;

mapping the partial feature points in the atrium to be modeled to the atrial template model by using an affine transformation network, thereby obtaining point cloud data mapped to the atrial template model;

inputting the point cloud data mapped to the atrial template model into a prediction network to predict displacements of the control feature points in the atrial template model; and

obtaining a three-dimensional anatomical model of the atrium to be modeled by deforming the atrial template model based on the displacement of the control feature points.

2. The method according to claim 1, wherein the displacements of the control feature points have a linear transformation relationship with displacements of mesh vertices in the atrial template model.

3. The method according to claim 1, wherein obtaining the point cloud data of the atrium to be modeled within the heart comprises:

obtaining positional coordinate information of partial feature points within the atrium to be modeled by using an electrode, thereby obtaining the point cloud data of the atrium to be modeled.

4. The method according to claim 1, wherein mapping the partial feature points in the atrium to be modeled to the atrial template model by using an affine transformation network, thereby obtaining the point cloud data mapped to the atrial template model, comprises:

normalizing the point cloud data of the atrium to be modeled;

inputting the normalized point cloud data into a fully-connected neural network, so as to predict an affine transformation matrix between the atrium to be modeled and the atrial template model; and

mapping the partial feature points in the atrium to be modeled to the atrial template model based on the predicted affine transformation matrix, thereby obtaining the point cloud data mapped to the atrial template model.

5. The method according to claim 4, wherein the fully-connected neural network is trained via a backpropagation algorithm using a first loss function defined by an expression:

$$L_1 = \| V_{init} - (T_1 V^T_{groundtruth})^T \|^2_2,$$

where: $L_1$ denotes a value of the first loss function, $V_{init}$ denotes a matrix corresponding to positional coordinate information of mesh vertices in the atrial template model, $T_1$ denotes an affine transformation matrix, $V_{groundtruth}$ denotes a matrix corresponding to coordinate ground truth values of mesh vertices in a three-dimensional network corresponding to the atrium to be modeled, and $\| \|^2_2$ denotes a squared Euclidean norm.

6. The method according to claim 1, wherein:

the prediction network comprises a plurality of point set abstraction module;
inputting the point cloud data mapped to the atrial template model into the prediction network to predict the displacements of the control feature points in the atrial template model comprises:

inputting the point cloud data mapped to the atrial template model into a first point set abstraction module,
iteratively inputting output data of each preceding point set abstraction module into a subsequent point set abstraction module in sequence,
and obtaining the predicted displacements of the control feature points in the atrial template model as output data from a last point set abstraction module;
wherein each point set abstraction module performs operations comprising:

randomly sampling k points from the input data and treating each sampled point as a centroid point;
for each centroid point, selecting m points within a spherical region having a specified radius centered at said each centroid point;
grouping the selected m sampled points corresponding to each centroid point into a point set;
extracting feature information for each point set and performing a max pooling operation to obtain global feature information corresponding to each sampled point; and
outputting the sampled k points, and the global feature information corresponding to each sampled point.

7. The method according to claim 6, wherein the prediction network is trained via a backpropagation algorithm using a second loss function defined by an expression:

$$L_2 = \|V_p - V_{groundtruth}\|^2_2,$$

where: $L_2$ denotes a value of the second loss function, $V_p$ denotes a matrix corresponding to coordinate prediction values of mesh vertices in a three-dimensional mesh model of the atrium to be modeled, $V_{groundtruth}$ denotes a matrix corresponding to coordinate ground truth values of mesh vertices in a three-dimensional anatomical model corresponding to the atrium to be modeled, and $\| \|^2_2$ denotes a squared Euclidean norm.

8. A three-dimensional cardiac mapping system, comprising:

an acquisition device, configured to obtain point cloud data of an atrium to be modeled within a heart;
a processor, configured to execute the method for constructing a three-dimensional cardiac anatomical model according to any one of claims 1 to 7, so as to output a three-dimensional anatomical model of the atrium to be modeled; and
a display device, configured to display the three-dimensional anatomical model of the atrium to be modeled.

9. The three-dimensional cardiac mapping system according to claim 8, wherein the acquisition device comprises at least one of an electrode catheter and an electrode patch.

10. A computer-readable storage medium, storing a program that, when executed by a processor, is capable of implementing the method according to any one of claims 1 to 7.

obtaining an atrial template model — 101

obtaining point cloud data of an atrium to be modeled within a heart — 102

mapping partial feature points in the atrium to be modeled to the atrial template model by using an affine transformation network — 103

inputting the point cloud data mapped to the atrial template model into a prediction network to predict displacements of the control feature points in the atrial template model; and deforming the atrial template model based on the displacements of the control feature points, thereby obtaining a three-dimensional anatomical model of the atrium to be modeled — 104

FIG. 1

affine transformed point cloud data

moved control feature points

point cloud data

STN  f

atrial template model and control feature points (black points)

Point Net++  P

deformed atrial template model

$T_I^{-1}$

3D anatomical model of the atrium to be modeled

FIG. 2

201 acquisition device → 202 processor → 203 display device

FIG. 3

# EP 4 648 017 A1

## INTERNATIONAL SEARCH REPORT

| | International application No. |
|---|---|
| | **PCT/CN2023/094488** |

### A. CLASSIFICATION OF SUBJECT MATTER

G06T17/00(2006.01)i; G06V10/82(2022.01)i

According to International Patent Classification (IPC) or to both national classification and IPC

### B. FIELDS SEARCHED

Minimum documentation searched (classification system followed by classification symbols)

G06T17/-,G06V10/-

Documentation searched other than minimum documentation to the extent that such documents are included in the fields searched

Electronic data base consulted during the international search (name of data base and, where practicable, search terms used)

CNABS, CNTXT, ENTXT, ENTXTC, VEN, CNKI, 互联网学术搜索, INTERNET ACADEMIC SEARCH: 心脏, 心房, 三维, 网格, 点云, 映射, 仿射变换, 绑定, 变形, 预测网络, 神经网络, 位移, 全连接, 矩阵, heart, atrium, cardiac, 3D, mesh, grid, point, cloud, map+, affin+, bound+, deform+, predict+, neural network, offset, displacement, FCN, matrix.

### C. DOCUMENTS CONSIDERED TO BE RELEVANT

| Category* | Citation of document, with indication, where appropriate, of the relevant passages | Relevant to claim No. |
|---|---|---|
| PX | CN 116129060 A (XINHANGLU MEDICAL TECHNOLOGY (GUANGZHOU) CO., LTD.) 16 May 2023 (2023-05-16)<br>claims 1-10 | 1-10 |
| Y | US 11475632 B1 (NEUCURES, INC.) 18 October 2022 (2022-10-18)<br>description, column 4, line 60 to column 5, line 30, and figure 4 | 1-3, 8-10 |
| Y | Marcel Beetz et al. "Point2Mesh-Net: Combining Point Cloud and Mesh-Based Deep Learning for Cardiac Shape Reconstruction"<br>*Statistical Atlases and Computational Models of the Heart*,<br>Vol. 13593, 28 January 2023 (2023-01-28), pages 280-290<br>ISSN: 978-3-031-23443-9,<br>sections 2.1-2.3 and figures 1-2 | 1-3, 8-10 |
| A | CN 111163692 A (NAVIX INTERNATIONAL LIMITED) 15 May 2020 (2020-05-15)<br>entire document | 1-10 |
| A | US 2020151952 A1 (ADOBE INC.) 14 May 2020 (2020-05-14)<br>entire document | 1-10 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **01 July 2023** | **08 August 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

11

## INTERNATIONAL SEARCH REPORT
### Information on patent family members

International application No.

**PCT/CN2023/094488**

| Patent document cited in search report | | | Publication date (day/month/year) | Patent family member(s) | | | Publication date (day/month/year) |
|---|---|---|---|---|---|---|---|
| CN | 116129060 | A | 16 May 2023 | CN | 116129060 | B | 23 June 2023 |
| US | 11475632 | B1 | 18 October 2022 | None | | | |
| CN | 111163692 | A | 15 May 2020 | WO | 2019034944 | A1 | 21 February 2019 |
| | | | | EP | 3668393 | A1 | 24 June 2020 |
| | | | | US | 2020289025 | A1 | 17 September 2020 |
| US | 2020151952 | A1 | 14 May 2020 | US | 10916054 | B2 | 09 February 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LORENSEN, W.E.** ; **CLINE, H.E.** Marching cubes: A high resolution 3D surface construction algorithm. *Proceedings of the 14th annual conference on Computer graphics and interactive techniques.*, 1987
- **KONG, F.** ; **SHADDEN, S.C.** Learning Whole Heart Mesh Generation From Patient Images for Computational Simulations. *IEEE Transactions on Medical Imaging*, 2022, vol. 42, 533-545
- **QI, C.** ; **YI, L.** ; **SU, H.** ; **GUIBAS, L.J.** PointNet++: Deep Hierarchical Feature Learning on Point Sets in a Metric Space. *NIPS*, 2017
- **QI, C.** ; **SU, H.** ; **MO, K.** ; **GUIBAS, L.J.** PointNet: Deep Learning on Point Sets for 3D Classification and Segmentation. *2017 IEEE Conference on Computer Vision and Pattern Recognition (CVPR)*, 2016, 77-85